# EUROPÄISCHE PATENTSCHRIFT

(11) **EP 0 625 499 B1**
(45) Veröffentlichungstag und Bekanntmachung des Hinweises auf die Patenterteilung: **26.08.1998**
(21) Anmeldenummer: 94107477.5
(22) Anmeldetag: 13.05.1994
(51) Int. Cl.: C07C 51/10, C07C 65/11

(54) **Verfahren zur Herstellung von 2-Hydroxy-naphthalin-6-carbonsäure**
Process for the preparation of 2-hydroxynaphthalene-6-carboxylic acid
Procédé de préparation d'acide 2-hydroxynaphtalène-6-carboxylique

(30) Priorität: 21.05.1993 DE 4316932
(43) Veröffentlichungstag der Anmeldung: 23.11.1994
(73) Patentinhaber: HOECHST AKTIENGESELLSCHAFT, 65929 Frankfurt am Main (DE)
(72) Erfinder: Rittner, Siegbert, Dr., D-64546 Mörfelden (DE); Rüffer, Hans-Martin, Dr., D-65719 Hofheim (DE); Schmid, Jörg, Dr., D-65817 Eppstein (DE); Wisser, Thomas, Dr., D-65552 Limburg (DE)

(56) Entgegenhaltungen:
- DE-A- 2 837 053
- DE-C- 955 598

## Beschreibung

Die Erfindung betrifft ein Verfahren zur Herstellung von 2-Hydroxy-naphthalin-6-carbonsäure bzw. deren Disalz durch Umsetzung von Alkalimetall-β-naphtholat mit Kohlenmonoxid und einem Alkalimetallcarbonat.

2-Hydroxy-naphthalin-6-carbonsäure ist ein wichtiges Zwischenprodukt z. B. bei der Herstellung von Farbstoffen, Polyestern, Arzneimitteln und Textilhilfsmitteln (siehe beispielsweise EP-A 0 292 955 und US 4,393,191).

Technisch erfolgt die Herstellung dieser Verbindung nach der Kolbe-Schmitt-Reaktion, d. h. durch Umsetzung des entsprechenden β-Naphtholats mit Kohlendioxid (siehe z. B. EP-A 0 254 596 bzw. US 5,011,984). Die Ausbeuten dieses Verfahrens sind jedoch noch verbesserungsfähig.

Ein alternatives Herstellungsverfahren für aromatische Hydroxycarbonsäuren ist u. a. in GB 1 155 776 (≙ US 3,655 744) beschrieben. Dabei werden bestimmte aromatische Alkali- oder Erdalkalinaphtholate mit Alkali- oder Erdalkalicarbonaten, -carboxylaten oder -dicarboxylaten in Gegenwart von Kohlenmonoxid umgesetzt.
Ausbeuten und insbesondere die Produkt-Selektivität des dort beschriebenen Verfahrens sind jedoch nicht für alle Bereiche ausreichend.

In der WO 91/11422 ist ein Verfahren zur Herstellung von 2-Hydroxy-naphthalin-6-carbonsäure aus Kalium-β-naphtholat, Kaliumcarbonat und CO beschrieben, wobei die Reaktion in einer Kaliumformiatschmelze durchgeführt wird.

Obwohl nach diesem Verfahren, im Gegensatz zu dem in GB 1 155 776 beschriebenen, überwiegend die gewünschte 2-Hydroxy-naphthalin-6-carbonsäure entsteht, werden auch Nebenprodukte, wie 2-Hydroxy-naphthalin-3-carbonsäure und 2-Hydroxy-naphthalin-3,6-dicarbonsäure gebildet; daher bleibt Raum für weitere Verbesserungen.
Es wurde nun überraschend gefunden, daß sich 2-Hydroxy-naphthalin-6-carbonsäure, 2-Hydroxy-naphthalin-3-carbonsäure, 2-Hydroxy-naphthalin-3,6-dicarbonsäure und β-Naphthol bzw. deren Salze durch Umsetzung mit CO und Carbonat bei erhöhtem Druck und erhöhter Temperatur ineinander umwandeln lassen. Dabei stellte sich heraus, daß die Zusammensetzung des Ausgangsgemisches auf die Produktverteilung nur einen geringen Einfluß hat.

Die Möglichkeit einer solchen Umwandlung bei der Bildung von 2-Hydroxy-naphthalin-6-carbonsäure ist bisher lediglich unter den Bedingungen der Kolbe-Schmitt-Reaktion beschrieben worden (siehe z. B. US 4,345,094 und US 4,329,494).

Gegenstand der Erfindung ist daher ein Verfahren zur Herstellung von 2-Hydroxy-naphthalin-6-carbonsäure unter Verwendung von Kaliumcarbonat und CO bei Temperaturen oberhalb von 150°C und einem Druck von mindestens 10 bar, dadurch gekennzeichnet, daß 2-Hydroxy-naphthalin-3-carbonsäure und/oder 2-Hydroxy-naphthalin-3,6-dicarbonsäure bzw. deren Kaliumsalze und gegebenenfalls Kalium-β-naphtholat als Edukte verwendet werden.

Das erfindungsgemäße Verfahren erlaubt es, durch Wiedereinsatz der Nebenprodukte die Produktselektivität bezüglich 2-Hydroxy-naphthalin-6-carbonsäure erheblich zu steigern.

Die nach dem erfindungsgemäßen Verfahren eingesetzten Verbindungen 2-Hydroxy-naphthalin-3-carbonsäure und/oder 2-Hydroxy-naphthalin-3,6-dicarbonsäure bzw. deren Kaliumsalze stammen vorteilhafterweise aus der Produktion von 2-Hydroxy-naphthalin-6-carbonsäure, wo sie als Nebenprodukte auftreten.
Die Abtrennung der erfindungsgemäß eingesetzten Nebenprodukte von der 2-Hydroxy-naphthalin-6-carbonsäure kann dabei in gewohnter Weise durch Aufnahme des alkalischen Reaktionsproduktes in Wasser und anschließende selektive Fällung der Produkte mittels Mineralsäuren erfolgen. Gemäß der unterschiedlichen Säurestärken läßt sich das unumgesetzte β-Naphthol bereits bei neutralem pH-Wert abtrennen, die 2-Hydroxy-naphthalin-6-carbonsäure analog bei pH 4 und die übrigen Hydroxy- bzw.
Hydroxynaphthalindicarbonsäuren bei pH-Werten um 1. Eine Feinreinigung der 2-Hydroxy-naphthalin-6-carbonsäure erfolgt gemäß den bekannten Verfahrensweisen, beispielsweise durch Druckumlösung in Wasser oder die vorteilhaftere Reinigung mit Hilfe von 1,4-Dioxan.

Die abgetrennten Hydroxy-naphthalincarbonsäuren können vereinigt und durch Umsetzen mit KOH oder K₂CO₃, analog der Herstellung des Kalium-β-naphtholats, in ihre Kaliumsalze überführt und getrocknet werden. Nach Ergänzung mit einer der abgetrennten 2-Hydroxy-naphthalin-6-carbonsäure-Menge entsprechenden Kalium-β-naphtholat-Menge und den auf unspezifische Nebenreaktionen (Harzbildung) zurückzuführenden geringen Verlusten steht ein geeignetes Ausgangsprodukt für das erfindungsgemäße Verfahren zur Verfügung.

Das erfindungsgemäße Verfahren wird nach Methoden durchgeführt, wie sie beispielsweise in WO 91/11422 und in der deutschen Patentanmeldung mit dem Titel "Verfahren zur Herstellung von aromatischen Hydroxycarbonsäuren" oder auch allgemein in GB 1,155,776 beschrieben sind. In der deutschen Patentanmeldung wird ein Verfahren zur Herstellung von aromatischen Hydroxycarbonsäuren oder deren Salzen durch Umsetzung von Phenolaten bzw. Naphtholaten mit Alkalicarbonaten und Kohlenmonoxid vorgeschlagen, welches dadurch gekennzeichnet ist, daß die festen Edukte Alkalicarbonat und Alkaliphenolat oder Naphtholat in Form einer Dispersion in einer inerten organischen Flüssigkeit der Reaktion zugesetzt werden.

Das erfindungsgemäße Verfahren wird in Gegenwart von CO durchgeführt. Das CO kann als Gasatmosphäre über der Reaktionsmischung vorliegen oder über, bzw. auch direkt in die Mischung gedrückt werden. Um die Reaktion erfolgreich ablaufen zu lassen, werden mindestens stöchiometrische Mengen, bezogen auf das β-Naphtholat, an CO benötigt.

Das erfindungsgemäße Verfahren wird bei einem Druck von 10 bis 150 bar, vorzugsweise 10 bis 100 bar und besonders bevorzugt bei 10 bis 30 bar betrieben, wobei der Druck bei der Reaktionstemperatur gemeint ist.

Es kann technisches Kohlenmonoxid verwendet werden, d. h., geringe Mengen an Fremdgasen, wie N₂, CH₄, CO₂, H₂, sind unkritisch.

Die Reaktionstemperatur kann je nach Eigenschaften der Edukte, Produkte und Löse- oder Dispergiermittel in weiten Grenzen variiert werden. Im allgemeinen wird bei Temperaturen von 150 bis 400°C, bevorzugt von 200 bis 350°C, ganz besonders bevorzugt von 250 bzw. 260 bis 350°C gearbeitet.
Die Reaktionsdauer beträgt vorzugsweise zwischen 1 und 40 Stunden.

Zur Durchführung des erfindungsgemäßen Verfahrens können unterschiedliche apparative Ausführungsformen zum Einsatz gelangen, so kann beispielsweise als Reaktor ein Druckgefäß oder Kneter verwendet werden, der mit einem zur Durchmischung und CO-Begasung effizienten Rührorgan ausgerüstet und mit einer Dispersionseinspeisung verbunden ist. Dabei kann die Dispersion entweder getaktet zugepumpt oder zugedrückt bzw. kontinuierlich eindosiert werden.

Das erfindungsgemäße Verfahren kann diskontinuierlich, semikontinuierlich oder kontinuierlich betrieben werden.

Das erfindungsgemäß verwendete Kaliumcarbonat sollte vorzugsweise wenig Feuchtigkeit enthalten, insbesondere weniger als 0,5 Gew.-% Wasser. Eine geringe Menge von Feuchtigkeit ist aber nicht kritisch.

Bezogen auf das eingesetzte β-Naphtholat sollten mindestens äquivalenten Mengen an Carbonat eingesetzt werden. Im allgemeinen beträgt das stöchiometriche Verhältnis von β-Naphtholat zu Carbonat 1:1 bis 1:4, vorteilhafterweise liegt es bei 1:1 bis 1:3, bevorzugt bei 1:1 bis 1:1,5.

Die erfindungsgemäß verwendeten Löse- und Dispergiermittel sind unter den Reaktionsbedingungen inerte, temperaturstabile, flüssige Stoffe oder Stoffgemische, wie aliphatische, alicyclische oder aromatische Kohlenwasserstoffe aus der Erdöldestillation. Insbesondere geeignet sind Leichtöl, Schweröl, vorzugsweise Kerosin, Aromaten und deren Alkylderivate, wie Toluol, Xylol, Isopropyl- oder Diisopropylnaphthalin, Diphenyl, Alkyldiphenyle, Triphenyl und Alkyltriphenyle, sowie aliphatische und aromatische Etherverbindungen und deren Alkylderivate, wie Diphenylether, Anisol, Dicyclohexylether, und Mischungen aus denselben. Die Dispergiermittel stören die Reaktion nicht und können aus dem Reaktor abdestilliert werden oder im Anschluß an die Reaktion durch Dekantieren oder Destillieren von der Reaktionsmasse abgetrennt werden. Sie haben den Vorteil, daß sie nach Entfernung von möglicherweise gelösten oder mitgeschleppten Bestandteilen, wie β-Naphthol oder Wasser, zur Erzeugung frischer Dispersionen wiedereingesetzt werden können.

Es ist auch möglich, wie in WO 91/11422 beschrieben, der Reaktionsmischung Kaliumformiat zuzusetzen, das unter den Reaktionsbedingungen als klare, dünnflüssige Schmelze vorliegt.

Die verwendete Menge an Kaliumformiat ist unkritisch und kann in weiten Grenzen variieren. Vorteilhaft ist es jedoch, Kaliumformiat in größeren Menge als Lösemittel (Verdünnungsmittel) einzusetzen, wie beispielsweise in der 2,5-bis 18-fachen, bevorzugt in der 6- bis 15-fachen, Gewichtsmenge des eingesetzten Kalium-β-naphtholats.

Die Isolierung der erfindungsgemäß hergestellten 2-Hydroxy-naphthalin-6-carbonsäure kann auf die bei der Gewinnung der Edukte beschriebene Weise erfolgen.

Die nach dem erfindungsgemäßen Verfahren hergestellte 2-Hydroxy-naphthalin-6-carbonsäure ist ein wichtiges Zwischenprodukt zur Herstellung von Polyestern, Azofarbstoffen und Arzneimitteln.

Insbesondere stellt sie nicht nur einen wertvollen Synthesebaustein für Farbstoffe, Textilhilfsmittel und Arzneimittel dar (siehe z. B. EP-A 0 292 955 A), sondern auch ein wichtiges Monomeres zur Herstellung von flüssigkristallinen Polymeren mit überragenden Eigenschaften (siehe z. B. US-PS 4 393 191).

Die nachfolgenden Beispiele dienen zur Erläuterung der vorstehend beschriebenen Erfindung. Die Teile sind Gewichtsteile, die Prozentangaben stellen Gewichtsprozente dar, sofern nicht anders vermerkt. Die Gewichtsteile verhalten sich zu Volumenteilen wie Kilogramm zu Liter.

### Beispiele

### Beispiel 1

1 Teil 2-Hydroxy-naphthalin-3-carbonsäure wird unter Rühren und Heizen zusammen mit 0,60 Teilen 50 %iger Kalilauge in einer Druck-Apparatur mit Destillationsbrücke und Azeotropabscheider in 2 Teilen Toluol aufgeschlämmt. Das Wasser wird bis zur vollständigen Trockne abdestilliert und das Dikaliumsalz der 2-Hydroxy-naphthalin-3-carbonsäure isoliert.

14 Teile 2-Hydroxy-naphthalin-3-carbonsäure-dikaliumsalz werden zusammen mit 7,4 Teilen Kaliumcarbonat und 75 Teilen Kaliumformiat in einen Druckreaktor gegeben und bei 230°C von Restfeuchte befreit. Sodann werden 95 bar Kohlenmonoxid aufgedrückt und 4 Stunden unter Rühren bei 280°C gehalten.

Nach Abkühlen wird der Reaktionsansatz in Wasser aufgenommen und durch selektive Ansäuerung in üblicher Weise aufgearbeitet. Die Ausbeuten (bezogen auf 2-Hydroxy-naphthalin-3-carbonsäure im Einsatz) betragen: 47 % 2-Hydroxy-naphthalin-6-carbonsäure, 7,2 % unumgesetzte 2-Hydroxy-naphthalin-3-carbonsäure, 18 % β-Naphthol, 17 % 2-Hydroxy-naphthalin-3,6-dicarbonsäure.

### Beispiel 2

2-Hydroxy-naphthalin-3,6-dicarbonsäure-Trikaliumsalz wird analog Beispiel 1 dargestellt. Die Reaktion erfolgt ebenfalls analog Beispiel 1, wobei anstelle der 14 Teile 2-Hydroxy-naphthalin-3-carbonsäure 18 Teile 2-Hydroxy-naphthalin-3,6-dicarbonsäure-Trikaliumsalz einsetzt werden und die Reaktionszeit 5 Stunden betrug. Die Ausbeuten bezogen auf Einsatzprodukt betragen: 41 % 2-Hydroxy-naphthalin-6-carbonsäure, 7,1 % 2-Hydroxy-naphthalin-3-carbonsäure, 11 %
β-Naphthol, 28 % 2-Hydroxy-naphthalin-3,6-dicarbonsäure.

### Beispiel 3

Ein 3-L-Edelstahl-Druckautoklav wird mit 100 Teilen Kaliumformiat befüllt und die Schmelze bei 230°C unter Rühren im Vakuum von Restfeuchte befreit. Danach wird auf 280°C aufgeheizt und 50 bar Kohlenmonoxid aufgedrückt. Über eine Dosierpumpe wird im Verlauf von 5 Stunden eine Mischung von 120 Teilen β-Kaliumnaphtholat und 10 Teilen Dikaliumsalz der 2-Hydroxy-naphthalin-3-carbonsäure mit 25 Teilen Kaliumcarbonat als Suspension in Kerosin eindosiert. Nach Aufarbeitung des Reaktionsansatzes erhält man die 2-Hydroxy-naphthalin-6-carbonsäure in einer Ausbeute von 68 %, bezogen auf die eingesetzten Naphtholkomponenten. Daneben fallen 10 % unumgesetztes β-Naphthol, 4 % 2-Hydroxy-naphthalin-3-carbonsäure und 12 % 2-Hydroxy-naphthalin-3,6-dicarbonsäure an.

## Patentansprüche

1. Verfahren zur Herstellung von 2-Hydroxy-naphthalin-6-carbonsäure unter Verwendung von Kaliumcarbonat und CO bei Temperaturen oberhalb von 150°C und einem Druck von mindestens 10 bar, dadurch gekennzeichnet, daß 2-Hydroxy-naphthalin-3-carbonsäure und/oder 2-Hydroxy-naphthalin-3,6-dicarbonsäure bzw. deren Kaliumsalze und gegebenenfalls Kalium-β-naphtholat als Edukte verwendet werden.

2. Verfahren nach Anspruch 1, dadurch gekennzeichnet, daß die eingesetzten Naphthalincarbonsäuren als Nebenprodukte aus der Kolbe-Schmitt-Reaktion erhältlich sind.

## Claims

1. A process for the preparation of 2-hydroxynaphthalene-6-carboxylic acid using potassium carbonate and CO at temperatures above 150°C and at a pressure of at least 10 bar, which comprises using 2-hydroxynaphthalene-3-carboxylic acid and/or 2-hydroxynaphthalene-3,6-dicarboxylic acid and/or their potassium salts and, if desired, potassium β-naphtholate as starting materials.

2. The process as claimed in claim 1, wherein the naphthalenecarboxylic acids employed can be obtained as secondary products from the Kolbe-Schmitt reaction.

## Revendications

1. Procédé pour la fabrication d'acide 2-hydroxy-naphtalène-6-carboxylique, faisant usage de carbonate de potassium et de CO à des températures au-delà de 150°C et à une pression d'au moins 10 bars, caractérisé en ce qu'on utilise de l'acide 2-hydroxy-naphtalène-3-carboxylique et/ou de l'acide 2-hydroxy-naphtalène-3,6-dicarboxylique ou leurs sels de potassium, et éventuellement du β-naphtolate de potassium en tant qu'éduits.

2. Procédé selon la revendication 1, caractérisé en ce que les acides naphtalène-carboxyliques utilisés peuvent être obtenus en tant que sous-produits de la réaction de Kolbe-Schmitt.
